# FASCICULE DE BREVET EUROPEEN

(11) **EP 2 854 793 B1**
(45) Date de publication et mention de la délivrance du brevet: **27.10.2021**
(21) Numéro de dépôt: 13726204.4
(22) Date de dépôt: 30.05.2013
(51) Int. Cl.: A61K 9/51, A61K 9/107, A61K 9/06, A61K 9/00, A61K 47/14, A61K 31/402, A61K 31/203, A61K 47/24

(54) **NANOCAPSULES LIPIDIQUES COMPRENANT UN RÉTINOIDE, NANODISPERSION ET COMPOSITION LES CONTENANT, LEUR PROCÉDÉ DE PRÉPARATION ET LEUR UTILISATION EN DERMATOLOGIE**
LIPIDNANOKAPSELN MIT EINEM RETINOID, NANODISPERSION UND ZUSAMMENSETZUNG DAMIT, VERFAHREN ZUR HERSTELLUNG DAVON UND VERWENDUNG DAVON IN DER DERMATOLOGIE
LIPID NANOCAPSULES COMPRISING A RETINOID, NANODISPERSION AND COMPOSITION CONTAINING SAME, METHOD OF PRODUCING SAME AND USE THEREOF IN DERMATOLOGY

(30) Priorité: 01.06.2012 FR 1255109; 01.06.2012 US 201261654718 P
(43) Date de publication de la demande: 08.04.2015
(73) Titulaire: Galderma Research & Development, 06410 Biot (FR)
(72) Inventeur: MALLARD, Claire, 06250 Mougins (FR)
(74) Mandataire: Nederlandsch Octrooibureau
(86) Numéro de dépôt international: PCT/EP2013/061189
(87) Numéro de publication internationale: WO 2013/178749

(56) Documents cités:
- WO-A1-2006/066978
- US-A1- 2005 048 088
- US-A1- 2007 134 276
- US-A1- 2008 193 393
- US-A1- 2009 258 065
- LIU ET AL: "Isotretinoin-loaded solid lipid nanoparticles with skin targeting for topical delivery", INTERNATIONAL JOURNAL OF PHARMACEUTICS, ELSEVIER BV, NL, vol. 328, no. 2, 12 décembre 2006 (2006-12-12), pages 191-195, XP005883011, ISSN: 0378-5173, DOI: 10.1016/J.IJPHARM.2006.08.007
- None

## Description

La présente invention se rapporte à des nanocapsules comprenant un principe actif irritant et plus particulièrement l'acide 3"-tert-butyl-4'-(2-hydroxy-ethoxy)-4"-pyrrolidin-1-yl-[1,1';3',1"]-terphenyl-4-carboxylique, le dit principe actif irritant étant présent sous forme solubilisée au sein des nanocapsules huileuses. L'invention porte aussi sur la nanodispersion composée des nanocapsules huileuses dispersées dans une phase aqueuse et sur la composition pharmaceutique comprenant au sein d'un véhicule pharmaceutiquement acceptable la nanodispersion. L'invention porte également sur leur procédé de préparation et la description divulgue l'utilisation de la composition pharmaceutique pour le traitement des affections dermatologiques, en particulier l'acné.

L'homme de l'art sait que l'activité de certains principes actifs pharmaceutique est indissociable d'une certaine irritation. Il est cependant indispensable de trouver des compositions permettant de maintenir l'activité biologique du principe actif tout en diminuant au maximum son caractère irritant. Les rétinoïdes sont des actifs couramment utilisés en dermatologie mais connus comme étant pour la plupart des principes actifs irritants, il est donc important, tout en maintenant l'activité pharmaceutique, d'améliorer la tolérance de cette famille de molécule anti acnéique.

Le brevet WO 2006/066978 décrit la synthèse de l'acide 3"-tert-butyl-4'-(2-hydroxy-ethoxy)-4"-pyrrolidin-1-yl-[1,1';3',1"]-terphenyl-4-carboxylique ainsi que son utilisation pharmaceutique.

L'art antérieur présente plusieurs brevets de formulation pour améliorer la tolérance des rétinoïdes par voie topique, en ajoutant des composés anti-irritants dans la composition.

La demanderesse a protégé dans le brevet FR 2 894 820 des formulations galéniques mettant en œuvre des anti-irritants comme l'allantoïne, l'EDTA, en association avec un rétinoïde particulier l'adapalène.

Dans le brevet WO 2006/037552, les inventeurs ajoutent des constituants dans la base formulaire comme l'interleukin-8 inhibitor pour agir sur le processus d'irritation.

Dans le WO 2005/079775, les inventeurs améliorent la tolérance des rétinoïdes par l'ajout d'idébénone ou un de ces dérivés.

Won et al, US 5,955,109 incorporent un rétinoïde dans des microsphères poreuses (Microsponge®) pour diminuer la libération du rétinoïde dans les couches de la peau, ce qui engendre une diminution du niveau d'irritation par un contrôle de la cinétique de libération de l'actif au travers de la peau.

Dans le brevet WO 2005/039532, les auteurs mettent en œuvre un rétinoïde dans une microémulsion huile dans eau dans le but d'améliorer la biodisponibilité. Cette microémulsion est composée d'un phospholipide et d'un hyaluronate de sodium ou d'acide hyaluronique modifié.

Saurat et coll dans le brevet FR 2 865 651, propose l'association d'un rétinoïde avec un ou plusieurs fragments de hyaluronate dans une formulation à usage dermatologique dans le cas de traitement pour lesquels il sera nécessaire d'améliorer l'état de la peau.

Cattaneo dans le brevet US 2005/0281886, présente dans son invention des microparticules et nanoparticules de chitosan contenant un rétinoïde. Ces microparticules et nanoparticules générées par un chitosan de haute viscosité diminuent l'effet irritant des rétinoïdes.

Il existe, dans l'art antérieur, de nombreux systèmes nanométriques et de nombreuses techniques de nanoencapsulation. L'art antérieur décrit notamment des nanocapsules polymériques utilisant des techniques d'évaporation de solvant organique solubilisant le polymère qui forme donc une paroi rigide de la nanocapsule. Mais la présence de solvant organique peut être une source d'intolérance, ou de problème de toxicité.

L'art antérieur, et notamment le brevet CN 1491551 présente des formulations de nanocapsules avec pour actif l'ivermectine en suspension dans l'eau et réalisées via les émulsions polymériques, au moyen d'un procédé sans solvant mettant en œuvre une polymérisation in-situ de monomère. Le principal inconvénient des techniques d'encapsulation par polymérisation in situ et en particulier polymérisation interfaciale est la génération de composés de faibles masses moléculaires suite à des risques de polymérisation incomplète. Des problèmes de toxicité peuvent donc être engendrés du fait de la présence de ces faibles masses.

Les brevets WO 01/64328 et WO 2011/036234 présentent des nanocapsules lipidiques contenant des phosphatidylcholines mais en association avec un co-surfactant hydrophile dérivé de polyéthylène glycol, nécessaire à la réalisation des nanocapsules. Le procédé de préparation revendiqué de telles nanocapsules se fait par température d'inversion de phase (procédé TIP), ce qui engendre l'utilisation de cycles de température dans le procédé. Ces techniques ne sont donc pas applicables à la plupart des rétinoides, car elles provoquent une dégradation de l'actif. Par ailleurs dans ces mêmes brevets, rien n'est suggéré quant à la tolérance et stabilité de l'actif, tel qu'un rétinoïde au sein de ces compositions.

Le problème que se propose de résoudre ici la présente invention, est donc de concevoir une composition stable physiquement et chimiquement, contenant de l'acide 3"-tert-butyl-4'-(2-hydroxy-ethoxy)-4"-pyrrolidin-1-yl-[1,1';3',1"]-terphenyl-4-carboxylique, pour le traitement de pathologies dermatologiques, particulièrement l'acné, ledit rétinoïde étant sous forme solubilisée, la composition selon l'invention devant améliorer la tolérance du principe actif tout en présentant une facilité d'utilisation et une cosméticité acceptable pour une application sur toutes les zones du corps pouvant être touchées par la pathologie.

Par stabilité physique selon l'invention, on entend une composition dont les propriétés physiques telles que les caractères organoleptiques, pH, viscosité sont stables au cours du temps et à différentes conditions de températures, 4°C, température ambiante, 40°C.

Par stabilité chimique selon l'invention, on entend une composition dans laquelle le principe actif est stable chimiquement au cours du temps et ce quelle que soit la condition de température : 4°C, température ambiante, 40°C.

Selon la présente invention, le rétinoïde doit se présenter sous une forme solubilisée dans une composition stable. De nombreux rétinoïdes présentent souvent des difficultés de solubilisation. Les rétinoïdes selon l'invention et notamment le rétinoïde préférentiellement utilisé, présentent une faible solubilité, limitant donc l'incorporation de ceux-ci dans les véhicules cités dans les brevets précédents, et rendant difficile l'obtention de composition stable. Par ailleurs, l'ajout d'agent solubilisant dans les formulations topiques augmente souvent le pouvoir irritant des formules.

Afin d'améliorer la tolérance de rétinoïdes irritants et la stabilité de l'actif dans une formulation aqueuse pour application cutanée, la demanderesse a découvert de manière surprenante qu'une composition permettant de modifier la structure de l'interface entre le milieu de solubilisation du rétinoïde et la phase aqueuse avait une influence sur la stabilité et la tolérance du principe actif au sein de la composition. Dans la présente invention, le rétinoïde est solubilisé dans la phase interne de nanocapsules lipidiques.

Par nanocapsules lipidiques, on entend un système nanovésiculaire de taille nanométrique c'est-à-dire de taille inférieure au micromètre constitué d'une enveloppe lipidique non polymérique solide ou semi-solide obtenue à partir d'au moins un surfactant choisi parmi les lécithines entourant un cœur huileux liquide ou semi-liquide à température ambiante.

Par température ambiante, on entend une température comprise entre 15 et 25°C.

Par cœur huileux, ou phase interne lipidique, on entend la phase interne des nanocapsules lipidiques contenant un solvant lipophile non miscible à l'eau.

La présente invention porte donc sur la formulation de nanocapsules lipidiques pouvant améliorer la tolérance cutanée de rétinoïde dans le traitement de pathologies dermatologiques, notamment l'acné.

Les nanocapsules lipidiques peuvent de plus permettre le ciblage de l'actif grâce à la mise en œuvre d'objets de très petites tailles.

A la différence des liposomes dont le cœur est aqueux, la phase interne des nanocapsules lipidiques de la présente invention est lipophile permettant la solubilisation de principes actifs hydrophobes en plus grande quantité. La solubilisation de principes actifs hydrophobes ne peut être possible avec les liposomes que par l'intermédiaire de la membrane généralement constituée de phospholipides.

La présente invention est un système de mise en œuvre de nanocapsules lipidiques sans utilisation de solvant organique de type alcoolique souvent utilisé pour la formation de l'enveloppe, limitant donc les risques de toxicité et d'intolérance et en particulier d'irritation. Selon la présente invention, la composition comprend des nanocapsules lipidiques et non des nanosphères lipidiques. Par opposition, les nanosphères lipidiques autrement appelées les nanoparticules solides lipides (dites SLN) sont des particules matricielles, i.e. dont la totalité de la masse est solide à température ambiante. Lorsque les nanosphères contiennent un principe actif pharmaceutiquement acceptable, celui-ci est finement dispersé ou solubilisé dans la matrice solide. Les nanocapsules lipidiques selon l'invention sont des particules dont le cœur est composé d'un ou plusieurs corps gras liquide(s) ou semi-liquide(s) à température ambiante dans lequel le principe actif est solubilisé, et dont l'enveloppe est de nature lipophile et non polymérique. En effet, les nanocapsules lipidiques selon l'invention ne nécessitent aucun polymère et donc pas de polymérisation in situ.

Par ailleurs, le procédé de préparation des nanocapsules lipidiques selon l'invention n'utilise pas de variations de cycles de température susceptible de dégrader l'actif.

La Demanderesse a donc, de façon surprenante, découvert que des compositions comprenant au moins un rétinoïde en tant que principe actif irritant sous forme solubilisée au sein de nanocapsules lipidiques dans un environnement hydrophile, ne nécessitant pas l'utilisation de polymère ou de solvant organique, garantissent la stabilité de l'actif et la bonne tolérance de la composition. Les compositions selon l'invention peuvent également favoriser la pénétration cutanée de l'actif, ce qui est utile dans le traitement d'affections dermatologiques, notamment l'acné.

La présente invention a donc pour premier objet des nanocapsules lipidiques comme décrites dans la revendication 1 constituées :
- d'au moins un rétinoïde en tant que principe actif irritant;
- d'au moins une phase interne huileuse dans laquelle le rétinoïde est solubilisé ;
- d'une enveloppe non polymérique obtenue à partir d'au moins un surfactant ; lesdites nanocapsules ne contenant pas de solvant organique de type alcoolique.

La présente invention a également pour objet une nanodispersion comme décrite dans la revendication 9, composée des nanocapsules dispersées dans une phase hydrophile.

Par nanodispersion, on entend donc le système lipidique composé des nanocapsules lipidiques à interface solide ou semi-solide dispersées dans une phase hydrophile continue, lesdites nanocapsules contenant une phase interne huileuse dans laquelle le principe actif irritant, et notamment le rétinoïde, est solubilisé, une enveloppe obtenue à partir d'un surfactant, formant l'interface semi-solide ou solide entre la phase interne huileuse et la phase hydrophile continue.

Ladite nanodispersion selon l'invention est incorporée dans un véhicule pharmaceutiquement acceptable, tel un gel, une solution ou une émulsion comme une crème ou une lotion.

La présente invention concerne donc également une composition décrite dans la revendication 10, notamment pharmaceutique, ladite composition comprenant, au sein d'un véhicule pharmaceutiquement acceptable, la nanodispersion selon l'invention.

La présente invention concerne donc une composition pharmaceutique, ladite composition comprenant au sein d'un véhicule pharmaceutiquement acceptable, la nanodispersion composée de nanocapsules lipidiques dispersées dans une phase hydrophile constituées:
- d'au moins un principe actif irritant, l'acide 3"-tert-butyl-4'-(2-hydroxy-ethoxy)-4"-pyrrolidin-1-yl-[1,1';3',1"]-terphenyl-4-carboxylique;
- d'au moins une phase interne huileuse dans laquelle le rétinoïde est solubilisé sans utilisation de solvant organique volatil tel que les alcools ;
- d'une enveloppe non polymérique obtenue à partir d'au moins un surfactant choisi parmi les lécithines, lesdites nanocapsules ne contenant pas de solvant organique de type alcoolique.

Par composition selon l'invention, on entend donc la nanodispersion, incorporée dans un véhicule pharmaceutiquement acceptable, tel un excipient ou un mélange d'excipients pouvant former une composition sous forme d'un gel, une solution ou une émulsion comme une crème ou une lotion pouvant être pulvérisable ou non

En particulier, la présente invention se rapporte donc à des nanocapsules lipidiques telles que décrites dans les revendications réalisées sans solvant organique de type alcoolique.

Par nanocapsules lipidiques selon la présente invention, on entend des nanosystèmes lipidiques présentant une taille moyenne inférieure au micromètre, de préférence inférieure à 800nm et de préférence inférieure à 500nm.

Les nanocapsules lipidiques sont présentes dans la composition selon l'invention en quantité comprise entre 0.1 et 30% en poids par rapport au poids total de la composition, de préférence entre 0.5 et 20%, et plus particulièrement entre 1 et 10%.

Les nanocapsules sont chacune constituées d'un cœur liquide ou semi-liquide à température ambiante contenant le principe actif, et d'une enveloppe obtenue à partir d'au moins un surfactant.

L'art antérieur (WO 01/64328 et WO2011/036234) présente des nanocapsules lipidiques contenant des phosphatidylcholines mais systématiquement en association avec un co-surfactant non-ionique hydrophile dérivé oxyéthyléné d'alcools gras et d'acides gras soit le 2-hydroxystéarate polyéthylène glycol vendu sous le nom de Solutol® HS 15 par la société BASF. A la différence de l'art antérieur, la présente invention porte sur des nanocapsules lipidiques contenant des phosphatidylcholines sans co-surfactant lipophile ou hydrophile.

L'enveloppe de surfactant encapsulant le cœur liquide ou semi-liquide à température ambiante est de préférence composée d'un matériau rigide à température ambiante non polymérique choisi parmi les lécithines et dont la température de transition ou de fusion est élevée. Pour être rigide à température ambiante, la température de transition ou de fusion doit être supérieure à 35°C, de préférence supérieure à 40°C et idéalement supérieure à 45°C.

Dans les nanocapsules selon l'invention, l'enveloppe est constituée par au moins un surfactant lipophile. Le surfactant est choisi parmi la famille des lécithines.

Les phosphatidylcholines montrent une bonne compatibilité avec la peau avec un très faible potentiel irritant.

Comme lécithines utilisables, on peut citer notamment les lécithines de soja ou d'œuf, naturelles ou synthétiques ou dérivés. Le premier type de lécithine est la phosphatidylcholine (PC). Il existe d'autres types de lécithine incluant le phosphatidylglycerol, le phosphatidylinositol, la sphingomyéline et la phosphatidyléthanolamine.

Parmi les lécithines présentant une température de transition supérieure à 35°C, on peut citer la dipalmitoylphosphatidylcholine (DPPC), la phosphatidylcholine distéaroyl (DSPC), la phosphatidylcholine dibehenyl (DBPC), palmitoyl-stéaroyl phosphatidylcholine (PSPC) palmitoyl-béhényle phosphatidylcholine (PSPC), stéaroyl-béhényle phosphatidylcholine (SBPC), ainsi que toutes lécithines saturées avec de longues chaines d'acides gras et leurs dérivés.

Les lécithines notamment utilisées dans la présente invention sont solides à température ambiante, ce qui favorise la formation d'une interface semi-solide autour du cœur liquide ou semi-liquide. Cette formulation permet l'encapsulation de l'actif solubilisé dans la phase interne, plus particulièrement le rétinoïde.

De préférence parmi la famille des lécithines présentant une température de transition élevée le surfactant lipophile est une lécithine hydrogénée, avantageusement dont le pourcentage de phosphatidylcholine saturée (ou hydrogénée) est élevé. Par pourcentage élevé, on entend une quantité supérieure à 85% de phosphatidylcholine saturée (ou hydrogénée) par rapport au poids total de lécithine.

Les nanocapsules lipidiques selon l'invention contiennent plus particulièrement une interface semi-solide ou solide entre la phase interne et la phase continue aqueuse, grâce à l'utilisation comme unique surfactant d'une lécithine dont le pourcentage de phosphatidylcholine saturée est élevé, ladite composition selon l'invention étant dépourvue de co-surfactant lipophile ou hydrophile.

Comme lécithine préférentiellement utilisées selon l'invention, on peut citer, certaines lécithines hydrogénées avec une teneur en phosphatidylcholine hydrogénée supérieure à 85%, comme par exemple le Lipoid® de grade S100-3 ou SPC-3, l'Epikuron® de grade 200 SH ou 100H, ou le Phospholipon® de grade 90H ou 100H.

Préférentiellement, la lécithine utilisée comme unique surfactant est le Phospholipon® 90H pour lequel la teneur en phosphatidylcholine hydrogénée est supérieure à 90% et comprenant 85% de phosphatidylcholine distéaroyl (DSPC) et 15% dipalmitoylphosphatidylcholine (DPPC), et dont la température de transition est environ 54°C

Le surfactant lipophile enveloppant le coeur liquide ou semi-liquide tel que défini ci-dessus est présent en quantité comprise entre 0.01 et 10% en poids, de préférence entre 0.05 et 5% en poids, et plus préférentiellement entre 0.1 et 1% en poids par rapport au poids total de la composition.

Le surfactant lipophile, notamment la lécithine hydrogénée, selon l'invention permet à elle seule l'encapsulation du rétinoïde, ce qui évite le contact de cet actif avec la phase hydrophile, et ainsi lui assure la stabilité chimique. En particulier, la composition, et notamment l'enveloppe, est exempte de co-surfactant lipophile ou hydrophile.

La composition selon l'invention comprend donc au sein des nanocapsules, au moins un principe actif connu de l'homme de l'art pour présenter un caractère irritant. Le principe actif utilisé dans l'invention est l'acide 3"-tert-butyl-4'-(2-hydroxy-ethoxy)-4"-pyrrolidin-1-yl-[1,1';3',1"]-terphenyl-4-carboxylique.

Dans la suite de la présente demande, l'acide 3"-tert-butyl-4'-(2-hydroxy-ethoxy)-4"-pyrrolidin-1-yl-[1,1';3',1 "]-terphenyl-4-carboxylique, composé préféré selon l'invention, sera également appelé composé A.

La composition comprend entre 0.00001 et 1% d'au moins un rétinoïde en poids par rapport au poids total de la composition, de préférence de 0.0001 à 0.5 % et de manière préférentielle, la composition selon l'invention contient de 0.001% à 0.05% d'un rétinoïde en poids par rapport au poids total de la composition. Dans un mode préféré selon l'invention, la composition comprend entre 0.001 et 0.05% d'acide 3"-tert-butyl-4'-(2-hydroxy-ethoxy)-4"-pyrrolidin-1-yl-[1,1';3',1"]-terphenyl-4-carboxylique, plus particulièrement entre 0.003 et 0.03 % en poids par rapport au poids total de la composition.

Le principe actif irritant, notamment le rétinoïde et plus particulièrement l'acide 3"-tert-butyl-4'-(2-hydroxy-ethoxy)-4"-pyrrolidin-1-yl-[1,1';3',1"]-terphenyl-4-carboxylique se trouve ainsi solubilisé dans le cœur des nanocapsules lipidiques selon l'invention. Ledit cœur, ou phase interne huileuse, comprend au moins un corps gras liquide ou semi-liquide à température ambiante.

La composition de la phase interne est donc essentielle pour la stabilité du principe actif. La phase interne huileuse doit bien entendu être compatible avec l'actif à solubiliser, et solubilisante de l'actif.

Par phase solubilisante de l'actif on entend une phase au sein de laquelle le principe actif présente une solubilité strictement supérieure à 0.1 %.

Cette phase interne huileuse comprend donc au moins un solvant huileux, choisi parmi les, les triglycérides et huiles en contenant, les huiles minérales, les esters d'acides gras, les acides gras polyéthoxylés, les alcools gras et esters correspondants, les glycols à l'exception des éthers de glycol.

Selon l'invention, la phase interne huileuse ne contient pas de solvants de type acides gras purs (i.e. sous la forme acide, non éthoxylé,).

Par solvant huileux, on entend toute matière non miscible à l'eau d'origine, naturelle, animale ou synthétique.

Parmi les triglycérides et huiles en contenant, on peut citer à titre non limitatif, les triglycérides d'acide octanoïque ou encore les triglycérides des acides caprylique/caprique comme ceux vendus par la société Stearineries Dubois ou ceux vendus sous la dénomination Miglyol® 810, 812, et 818 par la société Sasol.

Parmi les huiles minérales, on peut citer à titre non limitatif l'huile de paraffine.

Parmi les esters d'acide gras on peut citer à titre non limitatif le diisopropyl adipate, le cétéaryl isononanoate.

Parmi les alcools gras, on peut citer à titre non limitatif l'octyl dodécanol ou l'octanoate d'octyl dodécanol.

Dans un mode préféré selon l'invention, la phase interne huileuse préférée solvante du principe actif est le diisopropyl adipate, tel le produit commercial Crodamol® DA vendu par la société Croda.

En sus de ce(s) solvant(s) huileux, la phase interne peut également comprendre un ou plusieurs corps gras liquides ou semi-liquides à température ambiante non solubilisants de l'actif.

Par corps gras non solubilisant de l'actif, on entend un composé pour lequel le rétinoïde a une solubilité inférieure ou égale à 0.1%.

De même, la phase interne huileuse peut également contenir un ou plusieurs co-solvants non huileux, de type N-methyl-2-pyrrolidone, ou le diméthyl isosorbide, ou encore le diméthylsulfoxyde à l'exception des éthers de glycols.

Selon un mode préféré selon l'invention, le principe actif est solubilisé dans la phase interne huileuse en l'absence de tout solvant organique, type alcoolique tel l'éthanol, alors que ce dernier est généralement nécessaire à la solubilisation de principe actif tel les rétinoïdes (tel que décrit dans US 2005/0048088).

Dans la phase interne huileuse, le solvant préféré et plus particulièrement le diisopropyl adipate, sera présent en quantité comprise entre 50 et 99.997% en poids par rapport au poids total de la phase interne ; de préférence en quantité comprise entre 70 et 99.997% en poids par rapport au poids total de la phase interne, de préférence entre 95 et 99.997%.

Dans la phase interne huileuse, le corps gras ou co-solvant optionnel est présent en quantité comprise entre 0 et 50% en poids par rapport au poids total de la phase interne ; de préférence en quantité comprise entre 0,1 et 25% en poids par rapport au poids total de la phase interne, de préférence entre 0,5 et 10%.

Dans la nanodispersion selon l'invention, la phase interne huileuse des nanocapsules est présente en quantité comprise entre 0.1 et 50% en poids par rapport au poids total de la nanodispersion, de préférence en quantité comprise entre 0.5 et 30% en poids par rapport au poids total de la nanodispersion, plus préférentiellement entre 1 et 10%.

Dans la nanodispersion selon l'invention, le ratio entre la phase huileuse interne et la quantité de lécithine est compris entre 5 pour 1 et 10 pour 1. De manière préférée, ce ratio est compris entre 6 poru1 et à pour 1 et préférentiellement égal à 7 pour 1.

Au sein de la nanodispersion, les nanocapsules sont dispersées dans une phase hydrophile. La phase hydrophile continue comprend de l'eau. Cette eau peut être de l'eau déminéralisée, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains. L'eau peut être présente à une teneur comprise entre 55 et 95 % en poids par rapport au poids total de la composition, de préférence comprise entre 60 et 95 % en poids.

La présente invention a donc pour objet une composition, notamment pharmaceutique, ladite composition comprenant la nanodispersion contenant les nanocapsules lipidiques définies plus haut dans le texte de la présente invention au sein d'un véhicule pharmaceutiquement acceptable, tel un gel, une solution ou une émulsion comme une crème ou une lotion.

Lorsque le véhicule pharmaceutiquement acceptable est un gel, la nanodispersion est dispersée dans une phase hydrophile qui comprend au moins un agent gélifiant. Cet agent gélifiant est de préférence un dérivé cellulosique choisi parmi les gélifiants cellulosiques semi-synthétiques, tels que la méthylcellulose, l'éthylcellulose, l'hydroxypropylméthylcellulose, l'hydroxyéthylcellulose, la carboxyméthylcellulose, l'hydroxyméthylcellulose, et l'hydroxypropylcellulose, pris seuls ou en mélange. On utilise de préférence l'hydroxypropylméthylcellulose ou l'hydroxyéthylcellulose. Ces composés sont notamment commercialisés par la société Colorcon sous le nom de Methocel® (par exemple : Methocel® E4M) ou par la société Ashland sous le nom de Natrosol® (par exemple : Natrosol® 250 HHX). L'agent gélifiant peut également être choisi parmi les gommes naturelles comme la gomme de tragacanthe, la gomme de guar, la gomme d'acacia, la gomme arabique, la gomme xanthane, l'amidon et ses dérivés, les biopolymères tels que l'alginate de sodium, la pectine, la dextrine, le chitosan, le hyaluronate de sodium et leurs dérivés pris seuls ou en mélange. L'agent gélifiant peut être également choisi parmi les copolymères d'acide polyacrylique et de methylmethacrylate vendu sous le nom par exemple de Carbopol^{®} ou de Pemulen^{®} par la société Lubrizol ainsi que leurs copolymères tels que les Ultrez^{®} 10 ou 20, les carboxyvinyl polymères, les polyvinyl pyrrolidones et leurs dérivés, les polyvinyl alcools. L'agent gélifiant peut également être choisi parmi le composé Sepigel 305 constitué d'un mélange polyacrylamide / isoparaffine en C13-C14/ laureth-7, ou le Simulgel® 600PHA ou Sepineo® P600, à savoir le sodium acryloyldiméthyltaurate copolymère/ isohexadecane/polysorbate 80, ces deux produits étant commercialisés par la société Seppic.

L'agent gélifiant est utilisé notamment à une concentration comprise entre 0,1 et 10% en poids, de préférence entre 0,1 et 5% en poids par rapport au poids total de la composition.

Lorsque le véhicule pharmaceutiquement acceptable est une solution, la nanodispersion est dispersée au sein d'un véhicule composé d'une phase hydrophile ou aqueuse.

Par phase hydrophile qui constitue le véhicule pharmaceutiquement acceptable, on entend toute phase hydrophile telle que définie précédemment dans la présente invention.

Lorsque le véhicule pharmaceutiquement acceptable est une crème ou une lotion, la nanodispersion est dispersée au sein d'un véhicule composé d'une phase hydrophile et d'une phase grasse comprenant ou pas au moins un surfactant ou émulsionnant.

Dans le cas, des véhicules pharmaceutiques sous forme crème ou une lotion, la composition selon l'invention comprend donc une phase grasse. Cette phase grasse peut comprendre par exemple, les huiles végétales, minérales, animales ou synthétiques, des huiles de silicones, et leurs mélanges.

La phase grasse de l'invention comprend
- Une ou plusieurs huiles minérales, comme les huiles de paraffine de différentes viscosités comme le Marcol® 152, le Marcol ® 52 ou le Primol® 352 vendu par Univar,
- Une ou plusieurs huiles végétales parmi lesquelles on peut citer l'huile d'amande douce, l'huile de palme, l'huile de soja, l'huile de sésame, l'huile de tournesol, l'huile de ricin hydrogénée, l'huile de coprah,
- Une ou plusieurs huiles synthétiques parmi lesquelles on peut citer l'Apricot Kernel Oil PEG-6 ester (Labrafil® M1944CS), le propylène glycol laurate (Lauroglycol® FCC), le propylène glycol monocaprylate (Capryol® 90) fournis par Gattéfossé, des triglycérides comme par exemple les triglycérides d'eacides caprique / caprylique sous le nom de Miglyol^{®} 812 est vendu par la société Sasol), des esters tel que le C12-C15 Alkyl Benzoate (Crodamol^{®} AB fourni par Croda) ou le cétéaryl isononanoate comme le produit vendu sous le nom de Kollicream® Cl par la société BASF France, ainsi que le PPG-15 Stearyl Ether tel le Arlamol^{®} PS15E) ou le palmitate d'isopropyle comme le produit vendu sous le nom de Crodamol® IPP par la société Croda,

- Une ou plusieurs huiles animales parmi lesquelles on peut citer la lanoline, le squalène, l'huile de poisson, l'huile de vison avec comme dérivé le squalane vendu sous le nom Cosbiol® par la société Laserson.
- Une ou plusieurs huiles de silicone améliorant les propriétés de la formule à l'application, comme la Cyclomethicone (St-Cyclomethicone 5NF®) ou la Dimethicone (Q7 9120® Silicon fluid Fluid de viscosité de 2.10⁻⁵ m²/s à 1,25.10⁻² m²/s (20 cSt à 12500 cSt) de Dow Corning),
- un ou plusieurs épaississants de phase grasse de type alcool gras comme l'alcool cétylique (Crodacol C70 fourni par Croda/ Lanette 16 fourni par BASF mais aussi Kolliwax® CA fourni par BASF), l'alcool cétearylique (Crodacol 1618 fourni par Croda, Tego Alkanol® 1618 fourni par Evonik mais aussi le Kolliwax® CSA 50 fourni par BASF), l'alcool stéarylique (Crodacol® S95 fourni par Croda, Kolliwax® SA fourni par Cognis mais aussi Tego Alkanol® 18 fourni par Evonik) mais aussi l'alcool béhénique (Lanette® 22 fourni par BASF, Nacol® 22-98 fourni par Sasol mais aussi Behenyl Alcohol® 65 80 fourni par Nikko Chems) ou de type cire de Carnauba fourni par Baerlocher mais aussi la cire d'abeille vendu sous le nom de Cerabeil Blanchie DAB® fourni par Univar, le tribéhénate de glycéryle tel que le Compritol® 888 fourni par Gattéfossé. Dans ce cas, l'homme du métier adaptera la température de chauffage de la préparation en fonction de la présence ou non de ces solides.

Ainsi, la phase grasse de la crème ou la lotion selon l'invention peut être présente à une teneur comprise entre 1 et 95 % en poids par rapport au poids total de la composition, de préférence entre 5 et 85%, plus préférentiellement entre 15 et 50% en poids par rapport au poids total de la composition

De préférence, lorsque le véhicule de la composition selon l'invention est une crème ou une lotion, l'émulsion est sous la forme d'une émulsion huile dans eau (H/E). Cette émulsion peut ne pas comprendre ou comprendre au moins un agent émulsionnant.

Dans le cas d'une crème ou d'une lotion comprenant au moins un agent émulsionnant, les concentrations préférées sont comprises entre 0,001 et 20 % en poids par rapport au poids total de la composition. De préférence encore la concentration est comprise entre 1 et 15% et de manière préférée entre 3 et 11% en poids par rapport au poids total de la composition.

Le pouvoir émulsifiant des émulsionnants est étroitement lié à la polarité de la molécule. Cette polarité est définie par le HLB (Balance Hydrophile/Lipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des émulsionnants hydrophiles.

Les émulsionnants peuvent être classés, selon leur structure, sous les termes génériques "ioniques" (anioniques, cationiques, amphotères) ou "non ioniques". Les émulsionnants non ioniques sont des émulsionnants qui ne se dissocient pas en ions dans l'eau et sont donc insensibles aux variations de pH.

Les émulsionnants non ioniques sont particulièrement bien adaptés pour la préparation d'émulsions de type huile-dans-eau, objet de la présente invention. Ainsi, le système émulsionnant, composant de l'émulsion de l'invention, comprend au moins un émulsionnant non ionique, à fraction prédominante hydrophile, c'est-à-dire présentant une HLB élevée, supérieure à environ 10.

On peut citer, à titre d'exemple non limitatif des émulsionnants non ioniques présentant une HLB élevée, les esters de sorbitan tels que le POE(20) sorbitan monooléate, vendu sous le nom de Tween® 80 (HLB=15); le POE(20) sorbitan monostéarate vendu sous le nom de Tween® 60 (HLB=14.9) ; les éthers d'alcools gras tels que le POE (21) stéaryl ether (HLB= 15.5) vendu sous le nom de Brij® 721 par la société Croda, ou le ceteareth 20 vendu sous le nom de Eumulgin® B2 (HLB de 15,5) par la société BASF, les esters de polyoxyéthylène glycol tel que le glycéryl stéarate and PEG 100 stéarate vendu sous le nom de Arlacel® 165 FL (HLB=11) par la société Croda , le PEG 6 Stéarate et PEG 32 stéarate vendu sous le nom de Tefose® 1500 (HLB= 10) par la société Gateffossé, les sucroesters de haut HLB tel que le PEG 20 methyl glucose sesquistéarate vendu sous les noms de Glucamate® SSE20 (HLB=15) par la société Amerchol et le sucrose laurate vendu sous le nom de Surfhope C-1216® (HLB=16) et le sucrose stéarate vendu sous le nom de Surfhope® C-1811 (HLB=11) par la société Gattefossé. De préférence, lesdits émulsionnants non ioniques de haute HLB, présentent une HLB comprise entre 10 et 18.

On citera, comme exemples non limitatifs, les émulsionnants non ioniques de basse HLB (lipophiles), les esters de sorbitan, tels que le monostéarate de sorbitan (HLB = 4.7) vendu sous le nom de Span® 60 par la société Croda, les esters de glycérol tel que le monostéarate de glycerol vendu sous le nom de Kolliwax® GMS II (HLB=3.8) par la société BASF, les esters de polyéthylène glycol tel que le PEG-6 isostéarate vendu sous le nom de Olepal isostéarique® (HLB=8) par la société Gattefossé, les sucroesters de bas HLB tel que le methyl glucose sesquistéarate vendu sous le nom de Glucate SS® (HLB= 6). par la société Amerchol et le sucrose dilaurate vendu sous le nom de Surfhope® C-1205 (HLB=5) et le sucrose tristéarate vendu sous le nom de Surfhope® C-1803 (HLB=3) par la société Gattefossé.

De préférence, lesdits émulsionnants non ioniques présentant de faible HLB, présentent une HLB inférieure à 10.

Les émulsionnants non ioniques peuvent être utilisés seuls ou en mélange de deux ou plusieurs d'entre eux pour former le système émulsionnant composant la crème ou la lotion de l'invention.

De préférence, on utilisera, en tant que système émulsionnant un ou plusieurs couples " émulsionnant non ionique de haute HLB" / " émulsionnant non ionique de faible HLB", il pourra en particulier s'agir d'un système émulsionnant non ionique comprenant au moins un émulsionnant non ionique présentant une HLB supérieure à environ 10 et au moins un tensioactif non ionique présentant une HLB inférieure à environ 10.

Le ratio de chacun des deux émulsionnants formant le couple précité est déterminé le plus souvent par le calcul de la HLB requise de la phase grasse utilisée.

A titre d'émulsionnants préférés on peut citer des émulsionnants hydrophiles de type, Glycéryl Stéarate & PEG-100 Stéarate vendu sous le nom Arlace®l 165FL par la société Croda; le PEG 6 stéarate et PEG 32 stéarate vendu sous le nom de TEFOSE® 1500 par Gattefossé, le PEG 20 méthyl glucose sesquistéarate vendu sous le nom de Glucamate® SSE 20 par Amerchol, le polyoxyéthylène (21) stéaryl éther vendu sous le nom Brij® 721 par Croda, et le cétéareth 20 vendu sous le nom de Kolliphor® CS20 par BASF; des émulsionnants lipophiles de type méthyl glucose sesquistéarate tels que le Glucate® SS vendu par Lubrizol.

Les émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions H/E en s'adsorbant à l'interface eau-huile et en formant un film ou des couches lamellaires.

La crème ou la lotion selon l'invention comprend également une phase hydrophile ou phase aqueuse.

Par phase hydrophile qui constitue le véhicule pharmaceutiquement acceptable, seule ou au sein d'une émulsion, on entend toute phase hydrophile telle que définie précédemment dans la présente invention.

La composition selon l'invention pourra en outre contenir au sein de la nanodispersion ou de le véhicule pharmaceutiquement acceptable des additifs ou combinaisons d'additifs, tels que :
- des agents conservateurs;
- des agents propénétrants ;
- des agents stabilisants ;
- des agents humectants ;
- des agents régulateurs d'humidité ;
- des agents régulateurs de pH ;
- des agents modificateurs de pression osmotique ;
- des agents chélatants ;
- des filtres UV-A et UV-B ;
- et des antioxydants.

Parmi les propénétrants utilisables selon l'invention, on peut notamment citer les glycols comme par exemple le propylène glycol, les éthers de glycols, la N-méthyl-2-pyrrolidone, ou encore le diméthylsulfoxyde, à l'exception des éthers de glycol.

Parmi les conservateurs utilisables selon l'invention on peut notamment citer le méthyl parabène, le propyl parabène, le chlorure de benzalkonium, le phénoxyéthanol vendu sous le nom de Phenoxetol ® par Clariant, l'alcool benzylique vendu sous le nom d'alcool benzylique par Merck, le benzoate de sodium vendu sous le nom de Probenz® SP par Unipex, le sorbate de potassium vendu sous le nom de Sorbate de potassium par VWR, l'acide benzoique vendu sous le nom Acide benzoïque par VWR, le 2-Bromo-2-Nitropropane-1,3-Diol vendu sous le nom de Bronopol® par Jan Dekker International, la chlorhexidine vendu sous le nom de Chlorexidine digluconate 20% solution par Arnaud Pharmacie, le chlorocrésol et ses dérivés, l'alcool éthylique et la diazolidinylurée. Ces conservateurs peuvent être utilisés seul ou en association afin de protéger efficacement les formules contre toute contamination bactérienne.

Les conservateurs utilisés préférentiellement dans l'invention sont les méthyl parabène, propyl parabène, alcool benzylique, phénoxyethanol et sorbate de potassium. Ils peuvent être utilisés de 0.01% à 5% et préférentiellement de 0.05 à 2%.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée. Ces additifs peuvent être présents dans la composition de 0 à 95% en poids par rapport au poids total de la composition.

La composition selon l'invention comprend ainsi de préférence dans un véhicule pharmaceutiquement acceptable, en poids par rapport au poids total de la composition, des nanocapsules composés de :
a) 0.1 à 5% de surfactant choisi parmi les lécithines ;
b) 1 à 20% de corps gras liquide ou semi-liquide à température ambiante, de préférence des esters d'acide gras ;
c) entre 0,00001 et 0,1% d'au moins d'acide 3"-tert-butyl-4'-(2-hydroxy-ethoxy)-4"-pyrrolidin-1-yl-[1,1';3',1"]-terphenyl-4-carboxylique.

Dans un mode préféré selon l'invention, la composition comprend dans un véhicule pharmaceutiquement acceptable, en poids par rapport au poids total de la composition :
a) 0.1 à 5% de lécithine ;
b) 1 à 5% d'esters d'acide gras;
c) 0.001 à 0.03% d'acide 3"-tert-butyl-4'-(2-hydroxy-ethoxy)-4"-pyrrolidin-1-yl-[1,1';3',1"]-terphenyl-4-carboxylique.;

La composition pharmaceutique utilisable selon l'invention est destinée au traitement de la peau et peut être administrée par voie topique, parentérale ou orale.

Par voie orale, la composition pharmaceutique peut se présenter sous forme liquide, ou pâteuse, et plus particulièrement sous formes de gélules, de dragées, ou de sirops.

Par voie parentérale, la composition peut se présenter sous forme de suspensions pour perfusion ou pour injection.

De préférence, la composition est administrée par voie topique. Par voie topique, on entend une application sur la peau, les muqueuses, les cheveux ou le cuir chevelu.

Par voie topique, la composition peut se présenter sous forme liquide, ou pâteuse, et plus particulièrement sous forme de crèmes, de laits, de pommades, de tampons imbibés, de syndets, de lingettes, de gels, de sprays, de mousses, de lotions, de sticks, de shampoings, ou de bases lavantes.

L'invention a également pour objet une composition pour améliorer la tolérance d'un principe actif irritant, comprenant, au moins un principe actif irritant, caractérisée en ce qu'elle comprend, au sein d'un véhicule pharmaceutiquement acceptable, des nanocapsules lipidiques dispersées dans une phase hydrophile, lesdites nanocapsules lipidiques contenant une phase interne huileuse dans laquelle le principe actif irritant est solubilisé, et une enveloppe non polymérique obtenue à partir d'au moins un surfactant choisi parmi les lécithines, la dite composition ne contenant pas d' acides gras.

Dans un mode préféré selon l'invention, l'invention concerne également une composition pour améliorer la tolérance d'un principe actif irritant solubilisé, caractérisée en ce que le principe actif irritant est solubilisé en l'absence de co-solvant de type alcoolique tel l'éthanol.

L'invention a également pour objet un procédé de préparation des compositions comprenant au moins l'acide 3"-tert-butyl-4'-(2-hydroxy-ethoxy)-4"-pyrrolidin-1-yl-[1,1';3',1"] terphenyl-4-carboxylique ou composé A. Ce procédé fait appel à un Homogénéisateur Haute Pression (HHP). En particulier, le procédé selon l'invention n'utilise pas de Température d'Inversion de Phase (TIP) (utilisé notamment dans les brevets FR 2 805 761 et FR 2 840 531), et ne nécessite donc pas de cycle(s) de montée et de descente en température. En effet, le procédé selon l'invention s'effectue dans le HHP à froid ; le HHP ne nécessite donc pas de chauffage et de refroidissement successifs, et n'est pas thermo-régulé.

Le procédé peut se définir dans les différentes étapes de fabrications suivantes :
1 - Préparation de la nanodispersion.
   a) Solubilisation du principe actif :
      Dans un récipient adapté et à l'aide d'un barreau magnétique, le principe actif est solubilisé dans la phase huileuse interne ou cœur huileux, comprenant, entre autre l'huile solubilisante du principe actif.
      La phosphatidylcholine hydrogénée mise en œuvre est dispersée dans cette même phase huileuse chauffée à environ 75°C.
   b) Préparation de la phase aqueuse
      Dans un récipient adapté, la totalité de la phase aqueuse est chauffée à 75°C.
      Un conservateur ou autre additif peut être ajouté à cette phase.
   c) Mélange des phases et Pré-Homogénéisation
      Une fois les deux phases en température, celles-ci sont mélangées sous agitation (homogénéisation à l'Ultra-Turrax® minimum 2 minutes à 8000 rpm).
      Une fois cette pré-homogénéisation réalisée, la nanodispersion est introduite dans le HHP (Homogénéisateur Haute Pression).
   d) Homogénéisation Haute Pression
      L'utilisation d'un homogénéisateur haute pression nécessite de fixer le nombre de passages dans la chambre d'homogénéisation et la pression d'homogénéisation.
      Le procédé d'homogénéisation est alors appliqué :
         - minimum 500 bars jusqu'à 1000 bars de pression d'homogénéisation dans la chambre d'homogénéisation,
         - entre 5 et 10 passages dans la chambre d'homogénéisation.
      Au cours des passages dans la chambre d'homogénéisation, la nanodispersion n'est pas chauffée, le système HHP n'est pas contrôlé en température.
2 - Incorporation de la nanodispersion dans le véhicule pharmaceutiquement acceptable.

Dans le cas d'un gel, l'étape de gélification de la nanodispersion intervient en fin de fabrication après les différents passages dans l'HHP, au cours du refroidissement de la nanodispersion.

Une base gel est formulée pour diluer la nanodispersion, et ainsi obtenir une nanodispersion gélifiée afin de faciliter l'application sur la peau.

Dans le cas d'une solution, la nanodispersion peut ou pas être diluée dans une phase hydrophile.

Dans le cas d'une crème ou d'une lotion, la crème ou lotion est réalisée au préalable. Puis la nanodispersion est incorporée au véhicule finalisé.

Le procédé pourra être adapté par l'homme de l'art en fonction des différents ingrédients utilisés afin de maintenir la stabilité de la nanodispersion au sein de la composition finale.

La composition selon l'invention est utilisable comme médicament.

En particulier, l'invention a également pour objet une composition pour son utilisation dans le traitement des affections dermatologiques, notamment humaines, tel que définies ci-après.
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome T ;

Préférentiellement, la composition selon l'invention est une composition pour son utilisation dans le traitement de l'acné, les ichtyoses, les états ichtyosiformes, l'hyperkératose palmoplantaire ou le psoriasis.

De manière particulièrement préférée la composition selon l'invention comprendra le Composé A pour traiter l'acné, les ichtyoses, les états ichtyosiformes, l'hyperkératose palmoplantaire ou le psoriasis.

Il va être maintenant donné, à titre d'illustration et sans aucun caractère limitatif, diverses formulations de compositions comprenant un rétinoïde.

### Exemple 1 : Données de solubilité du composé A dans différentes phases huileuse

L'objectif de cette étude de préformulation est d'identifier des phases huileuses dans lesquelles le composé A présente une solubilité supérieure à 0.1% m/m et dans lesquelles il est stable chimiquement.

La stabilité de l'actif a été évaluée par chromatographie en phase liquide couplée à un détecteur UV (HPLC-UV).

| **Nom INCI (nom commercial)** | **Solubilité maximale (%m/m)** | **Stabilité** |
|---|---|---|
| Propylène glycol monocaprylate (Capryol® 90) | 0.802 | 6 mois TA /40°C |
| Propylène glycol monolaurate (Lauroglycol® FCC) | 0.296 | 6 mois TA / 40°C |
| Diisopropyl adipate (Crodamol® DA) | 0.297 | 6 mois TA / 40°C |
| Oléate de macrogol (Labrafil® M1944CS) | 0.156 | 6 mois TA / 40°C |
| Octyl dodécanol (Eutanol® G) | 0.137 | Instable |
| Propylène glycol dicaprylate/dicaprate (Myritol® PC) | 0.069 | Instable |
| Triglycérides d'acides caprylique / caprique (Miglyol®812N) | 0.019 | 6 mois TA /40°C |
| Huile d'amande douce | 0.011 | 6 mois TA /40°C |
| Huile minérale | 0.0001 | |

| | | |
|---|---|---|
| * : TA Température ambiante | | |

Suite aux résultats de cette étude de solubilité et de stabilité, le diisopropyl adipate semble un solvant adapté pour obtenir les concentrations désirées du composé A dans le véhicule pharmaceutiquement acceptable.

### Exemple 2: Composition de la nanodispersion contenant les nanocapsules lipidiques avant dilution dans le véhicule pharmaceutique choisi contenant le composé A

| **Ingrédients Nom INCI** | **Ingrédients Nom commercial** | **Teneur (% m/m)** |
|---|---|---|
| Phosphatidylcholine hydrogénée | Phospholipon 90H | 2.00 |
| Diisopropyl adipate | Crodamol DA | 13.77 |
| Méthyl parabène | Nipagin M | 0.20 |
| Eau purifiée | -- | 84.0 |
| composé A | -- | 0.0276 |

### Exemple 3 : Exemples de compositions de type gel selon l'invention contenant le composé A

Afin de réaliser des compositions de type gel selon l'invention, différentes quantités de la nanodispersion préparée selon l'exemple 2 ont été prélevées et diluées dans une base gel constituée de l'eau plus gélifiant.

Pour obtenir un gel à 0.01% en principe actif, 36.23% de la solution de nanocapsules lipidiques selon l'exemple 2 a été diluée dans la base gel.

Pour obtenir un gel à 0,003% en principe actif, 9,2% de la solution de nanocapsules lipidiques selon l'exemple 2 a été diluée dans la base gel.

Des exemples de compositions de type gel obtenues selon l'invention sont donc les suivantes, qualitativement et quantitativement :

| **Ingrédients Nom INCI** | **Ingrédients Nom commercial** | **Compositions (% m/m)** | | | | |
|---|---|---|---|---|---|---|
| | | **N°1** | **N°2** | **N°3** | **N°4** | **N°5** |
| composé A | -- | 0.01 | 0.003 | 0.01 | 0.003 | 0.01 |
| Diisopropyl adipate | Crodamol DA | 4.96 | 1.27 | 4.96 | 1.27 | 4.96 |
| Phosphatidylcholine hydrogénée | Phospholipon 90H | 0.72 | 0.18 | 0.72 | 0.18 | -- |
| | Lipoid S100-3 | -- | -- | -- | -- | 0.80 |
| Méthyl parabène | Nipagin N | 0.20 | 0.20 | 0.20 | 0.20 | 0.20 |
| Hydroxyethylcellulose | Natrosol 250 HHX | 0.80 | 0.80 | -- | -- | -- |
| Polyacrylamide (and) C13-14 Isoparaffin (and) Laureth-7 | Sepigel 305 | -- | -- | 1.0 | 1.0 | -- |
| Acrylamide, AMPS Copolymer Dispersion 40% / Isohexadecane | Simulgel 600PHA | -- | -- | -- | -- | 1.0 |
| Eau purifiée | -- | Qsp* 100 | Qsp 100 | Qsp 100 | Qsp 100 | Qsp 100 |

| | | | | | | |
|---|---|---|---|---|---|---|
| *Qsp : Quantité suffisante pour | | | | | | |

### Exemple 4 : Etudes de stabilité physique de la nanodispersion selon l'exemple 2

Les nanocapsules lipidiques de l'exemple 2 ont été mise en stabilité à différentes conditions : 4°C, température ambiante et 40°C. La taille de ces nanocapsules lipidiques a été suivie au cours de l'étude de stabilité par analyse granulométrique en utilisant le Zetasizer Nanoseries-Nano-ZS (Malvern)

Deux dilutions sont utilisées pour effectuer les analyses granulométriques :
1d : 10µl de la nanodispersion dans 15 ml d'eau distillée filtrée
2d : 1mL de 1d dans 5 mL d'eau distillée

La solution de nanocapsules lipidiques présente deux populations granulométriques dont la fraction majoritaire en nombre est de l'ordre de la centaine de nanomètre.

| **Granulométrie T0** | Taille (nm) | 132 | | | 650 | | |
|---|---|---|---|---|---|---|---|
| | CV% | 6.5 | | | 45 | | |
| | % en nombre | >99% | | | <1% | | |
| **Stabilité en Température** | | **4°C** | | **T ambiante** | | **40°C** | |
| **T 2mois** | Taille (nm) | 117 | 434 | NR* | | 154 | 794 |
| | CV% | 8 | 30 | NR | | 15 | 40 |
| **T 3 mois** | Taille (nm) | 126 | 617 | 136 | 526 | 138 | 540 |
| | CV % | 10 | 25 | 9 | 30 | 9 | 30 |

| | | | | | | | |
|---|---|---|---|---|---|---|---|
| NR : non réalisé | | | | | | | |

Après 3 mois de mise en stabilité de la nanodispersion et ce quelle que soit la température, la taille granulométrique des nanocapsules lipidiques reste stable par rapport au temps initial.

### Exemple 5 Etudes de stabilité chimique de l'actif au sein des compositions 1 et 2 selon l'exemple 3

Les compositions 1 et 2 selon l'exemple 3 ont été mise en stabilité à différentes conditions : 4°C, température ambiante et 40°C et ont été suivies pendant 6 à 7 mois. A chaque pointage de stabilité, le composé A a été dosé par HPLC-UV.

Composition n°1 (0.01% rétinoïde soit 0.1 mg/g)

| **T0** | mg/g | 0.091 | | |
|---|---|---|---|---|
| | % RDS | 1.3 | | |
| | % Titre attendu | 91 | | |
| **Stabilité à la température** | | **4°C** | **T ambiante** | **40°C** |
| **T 1mois** | mg/g | 0.091 | NR | 0.097 |
| | % RDS | 0.2 | -- | 0.8 |
| | % recouvrement | **100.0** | -- | **106.6** |
| **T 2mois** | mg/g | NR | NR | 0.091 |
| | % RDS | -- | -- | 2.0 |
| | % recouvrement | -- | -- | 100 |
| **T 3 mois** | mg/g | 0.098 | NR | 0.097 |
| | % RDS | 0.4 | -- | 0.1 |
| | % recouvrement | 107.7 | -- | **106.6** |
| **T 3.5 mois** | mg/g | NR | 0.094 | 0.091 |
| | % RDS | -- | 0.4 | 1.6 |
| | % recouvrement | -- | 104 | 100 |
| **T 7 mois** | mg/g | 0.096 | 0.093 | NR |
| | % RDS | 1.6 | 3.5 | -- |
| | % recouvrement | **105.5** | **102.2** | -- |

| | | | | |
|---|---|---|---|---|
| NR : non réalisé | | | | |

Composition n°2 (0.003% de rétinoïde soit 0.03 mg/g)

| **T0** | mg/g | 0.028 | | |
|---|---|---|---|---|
| | % RDS | 0.5 | | |
| | % Titre attendu | 94 | | |
| **Stabilité à la température** | | **4°C** | **T ambiante** | **40°C** |
| **T 1mois** | mg/g | 0.028 | NR | 0.028 |
| | % RDS | 0.1 | -- | 0.5 |
| | % recouvrement | **100.0** | -- | **100.0** |
| **T 3 mois** | mg/g | 0.028 | NR | 0.027 |
| | % RDS | 2.5 | -- | 1.0 |
| | % recouvrement | **100.0** | -- | **96.4** |
| **T 4 mois** | mg/g | 0.028 | NR | 0.027 |
| | % RDS | 2.5 | -- | 1.0 |
| | % recouvrement | 100 | -- | 96 |
| **T 4 mois** | mg/g | NR | 0.029 | 0.028 |
| | % RDS | -- | 0.4 | 0.3 |
| | % recouvrement | -- | **103.6** | **100.0** |
| **T 6 mois** | mg/g | 0.029 | NR | 0.027 |
| | % RDS | 0.0 | -- | 1.9 |
| | % recouvrement | **103.6** | -- | **96.4** |

| | | | | |
|---|---|---|---|---|
| NR : non réalisé | | | | |

Conclusion : Le composé A est stable chimiquement aux 3 conditions de températures : 4°C, température ambiante et 40°C dans les compositions gel selon l'invention jusqu'à 6 à 7 mois.

### Exemple 6 : Procédé de fabrication par HHP des compositions 1 et 2 de l'exemple 3

Pour préparer 300 g de formulation avec l'homogénéisateur haute pression (HHP) :
Préparation d'une solution à 0.2% de composé A dans du Crodamol DA.

Faire chauffer la quantité totale de la solution précédemment préparée : 41.40 g à environ 70-75°C

Incorporer petit à petit sous agitation (petite défloculeuse) la totalité du Phospholipon 90H : 6 g

Maintenir l'agitation jusqu'à obtention d'un mélange homogène.

En parallèle faire chauffer la quantité d'eau de la formulation : 252g, sous agitation faible, y solubiliser le méthyl parabène : 0.6g.

Une fois les deux phases homogènes et à la même température (70°C), incorporer la phase huileuse contenant le principe actif et le Phospholipon 90H dans l'eau sous agitation à l'Ultra-Turrax à 8000 rpm.

Maintenir cette agitation pendant 2 minutes.

Verser ce mélange dans l'HHP.

Allumer le manomètre et amorcer la circulation du mélange dans l'HHP, en veillant à séparer l'eau restant dans le circuit de l'appareil avant de récupérer la formulation pour re-circulation.

Une fois que le circuit de l'HHP contient le mélange, fixer la valeur de la pression souhaitée sur le manomètre : entre 700 et 900 bars.

Le nombre de passage est fixé à 10 avec recirculation du mélange obtenu à la sortie du HHP.

Une fois la solution de capsules lipidiques obtenue, on ajoute 36.24% de cette solution dans 63.76% de gel Natrosol pour obtenir la viscosité et la dilution souhaitées pour la formulation 1 de l'exemple 3 à 0.01% en actif.

Une fois la solution de capsules lipidiques obtenue, on ajoute 10.87% de cette solution dans 63.76% de gel Natrosol pour obtenir la viscosité et la dilution souhaitées pour la formulation 2 de l'exemple 2 à 0.003% en actif

### Exemple 7: Autres Formulations de type gel qui ne sont

### pas selon l'invention et qui contiennent de la trétinoïne.

| **Ingrédients** | **Compositions (%m/m)** | | | | |
|---|---|---|---|---|---|
| | **N°6** | **N°7** | **N°8** | **N°9** | **N°10** |
| Trétinoine | 0.01 | 0.05 | 0.01 | 0.05 | 0.01 |
| Triglycérides d'acides caprylique/caprique | 5.0 | 1.5 | 5.0 | 1.5 | 5.0 |
| Butyl hydroxytoluène | 0.10 | 0.10 | 0.10 | 0.10 | 0.10 |
| Phosphatidylcholine hydrogénée (Phospholipon 90H) | 0.72 | 0.22 | 0.72 | 0.22 | -- |
| Phosphatidylcholine hydrogénée (Lipoid S100-3) | -- | -- | -- | -- | 0.72 |
| Méthyl parabène | 0.20 | 0.20 | 0.20 | 0.20 | 0.20 |
| Hydroxyéthylcellulose (Natrosol 250HHX) | 0.80 | 0.80 | -- | -- | -- |
| Polyacrylamide (and) C13-14 Isoparaffin (and) Laureth-7 (Sepigel 305) | -- | -- | 0.80 | 0.80 | -- |
| Acrylamide, AMPS Copolymer Dispersion 40% / Isohexadecane (Simulgel 600PHA) | -- | -- | -- | -- | 0.70 |
| Eau purifiée | Qsp 100 | Qsp 100 | Qsp 100 | Qsp 100 | Qsp 100 |

### Exemple 8 : Exemples de compositions de type crème sans émulsionnant selon l'invention contenant le composé A

Afin de réaliser des compositions de type émulsion selon l'invention ne contenant pas d'agent émulsionnant, différentes quantités de la nanodispersion préparée selon l'exemple 2 ont été prélevées et diluées dans la crème préparée au préalable.

Pour obtenir une crème à 0.01% en principe actif, 36.23% de la solution de nanocapsules selon l'exemple 2 a été diluée dans la base crème.

Pour obtenir une crème à 0.003% en principe actif, 9.2% de la solution de nanocapsules selon l'exemple 2 a été diluée dans la base crème.

| **Ingrédients** | **Compositions (% m/m)** | | | | |
|---|---|---|---|---|---|
| | **N°11** | **N°12** | **N°13** | **N°14** | **N°15** |
| Composé A | 0.01 | 0.003 | 0.01 | 0.003 | 0.01 |
| Diisopropyl adipate (Crodamol DA) | 4.96 | 1.27 | 4.96 | 1.27 | 4.96 |
| Phosphatidylcholine hydrogénée) Phospholipon 90H | 0.72 | 0.18 | 0.72 | 0.18 | -- |
| Phosphatidylcholine hydrogénée) Lipoid S100-3 | -- | -- | -- | -- | 0.80 |
| Glycérine | 5.0 | 5.0 | 5.0 | 5.0 | 5.0 |
| Decaméthylpentacyclosiloxa ne (ST-Cyclomethicone 5NF) | 7.0 | 7.0 | 7.0 | 7.0 | 7.0 |
| Propylène Glycol | 6.0 | 6.0 | 6.0 | 6.0 | 6.0 |
| Méthyl parabène (Nipagin M) | 0.2 | 0.2 | 0.2 | 0.2 | 0.2 |
| Phénoxyéthanol | 1.0 | 1.0 | 1.0 | 1.0 | 1.0 |
| Polyacrylamide (and) C13-14 Isoparaffin (and) Laureth-7 (Sepigel 305) | -- | -- | 4.0 | 4.0 | -- |
| Acrylamide, AMPS Copolymer Dispersion 40% / Isohexadecane (Simulgel 600PHA) | 4.0 | 4.0 | -- | -- | 4.0 |
| Eau purifiée | Qsp 100 | Qsp 100 | Qsp 100 | Qsp 100 | Qsp 100 |

### Exemple 9 : Exemples de compositions de type crème contenant au moins un agent émulsionnant selon l'invention contenant le composé A

Afin de réaliser des compositions de type crème contenant au moins un agent émulsionnant selon l'invention, différentes quantités de la nanodispersion préparée selon l'exemple 2 ont été prélevées et diluées dans la crème réalisée au préalable.

Pour obtenir une crème à 0.01% en principe actif, 36% de la solution de nanocapsules lipidiques selon l'exemple 2 a été diluée dans l'émulsion.

Pour obtenir une crème à 0.003% en principe actif, 9% de la solution de nanocapsules lipidiques selon l'exemple 2 a été diluée dans la crème

| **Ingrédients** | **Compositions (% m/m)** | |
|---|---|---|
| | **N°16** | **N°17** |
| composé A | 0.01 | 0.003 |
| Diisopropyl adipate (Crodamol DA) | 4.96 | 1.24 |
| Phosphatidylcholine hydrogénée (Phospholipon 90H) | 0.72 | 0.18 |
| Decaméthylpentacyclosiloxane (ST-Cyclomethicone 5NF) | 13.0 | 13.0 |
| Perhydrosqualène | 6.0 | 6.0 |
| Glucamate SSE 20 | 3.5 | 3.5 |
| Glucate SS | 3.5 | 3.5 |
| Glycérine | 3.0 | 3.0 |
| Propylène Glycol | 4.0 | 4.0 |
| Carbomer (Carbopol Ultrez 20) | 0.40 | 0.40 |
| Méthyl parabène | 0.20 | 0.20 |
| Propyl parabène | 0.10 | 0.10 |
| EDTA | 0.10 | 0.10 |
| Triéthanolamine | Qsp pH 5.5 +/- 0.5 | Qsp pH 5.5+/-0.5 |
| Eau purifiée | Qsp 100 | Qsp 100 |

### Exemple 10 : Exemples de compositions de type lotion selon l'invention contenant le composé A

| **Ingrédients** | **Compositions (% m/m)** | |
|---|---|---|
| | **N°18** | **N°19** |
| composé A | 0.01 | 0.003 |
| Diisopropyl adipate (Crodamol DA) | 4.96 | 1.24 |
| Phosphatidylcholine hydrogénée Phospholipon 90H | 0.72 | 0.18 |
| Perhydrosqualène | 5.0 | 5.0 |
| Cetostearyl isononanoate | 5.0 | 5.0 |
| Steareth-21 (Brij 721) | 3.0 | 3.0 |
| Glycéryl stéarate and PEG-100 stearate (Arlacel 165FL) | 3.0 | 3.0 |
| Dipropylène Glycol | 3.0 | 3.0 |
| Acrylamide, AMPS Copolymer Dispersion 40% / Isohexadecane (Simulgel 600 PHA) | 1.40 | 1.40 |
| Methyl parabène | 0.15 | 0.15 |
| Propyl parabène | 0.10 | 0.10 |
| Triéthanolamine | Qsp pH 5.5 +/- 0.5 | Qsp pH 5.5 +/- 0.5 |
| Eau purifiée | Qsp 100 | Qsp 100 |

### Exemple 11 : Etude de tolérance: Evaluation de l'effet pro-inflammatoire des formulations après application unique sur l'Oreille de souris BALB/C.

Le but de l'étude est d'évaluer l'effet d'une composition selon l'invention, sur l'activité pro-inflammatoire liée aux rétinoïdes de façon générale.

Une application unique de 20µ! des produits testés a été administrée sur l'oreille des souris au jour 1. Des observations cliniques et mesures de l'épaisseur de l'oreille de souris, directement reliée à l'inflammation, sont mesurées du jour 2 au jour 12.

Il a été observé que le composé A en application topique unique, à 0.01% dans l'acétone, induisait un effet inflammatoire significatif avec une augmentation de l'épaisseur de l'oreille de souris de 59% versus l'acétone seule.

Par contre, incorporé à 0.01% dans la composition gel 1 de l'exemple 3, l'application topique unique du composé A induit une inflammation modérée de 40%, soit une diminution significative de l'inflammation de 32%.

### Exemple 12: Etude de tolérance cutanée chez le modèle de porc Göttingen

L'objectif de cette étude est d'évaluer la tolérance de la composition gel 1 de l'exemple 3 par applications répétées en utilisant le modèle de tolérance de porc Göttingen comparée à une formulation de référence du composé A dans laquelle l'actif est solubilisé et non encapsulé dans un gel hydroalcoolique de référence. Les deux formulations sont évaluées à la même concentration de 0.01%.

50µl de chaque formulation est appliquée sur le dos de l'animal pendant 4 semaines et chaque application est répétée 6 fois. Les réactions cutanées de type érythème et oedème sont évaluées toutes les semaines après 7 jours d'application selon une échelle d'indice variant de 0 à 5.0.

Les résultats sont les suivants :

| | **Moyenne hebdomadaire des indices** | | | |
|---|---|---|---|---|
| | **Semaine 1** | **Semaine 2** | **Semaine 3** | **Semaine 4** |
| **Formulation de référence** | 0.000 | 0.071 | 0.238 | 0.875 |
| **Composition 1 de l'exemple 3** | 0.000 | 0.000 | 0.000 | 0.146 |

Après 4 semaines d'application, il a été observé que la composition 1 de l'exemple 3 est 6 fois moins irritante que la formulation de référence dans laquelle le composé A est solubilisé mais non encapsulé.

### Exemple 13: Etude d'activité comédolytique

L'activité comédolytique de la composition 2 de l'exemple 3 a été analysée par la mesure de la réduction de nombre de comédons, après application topique sur le dos de souris Rhino tous les jours pendant 11 jours versus placébo.

50 µl de la composition 2 de l'exemple 2 à 0.003% en composé A sont appliqués.

Après 11 jours d'application, une diminution significative du nombre de comédons de 70% est observée avec la composition selon l'invention versus 2% pour le placébo.

Cette étude démontre donc l'activité de la composition selon l'invention pour le traitement de l'acné. L'efficacité avérée de la composition selon l'invention démontre, que malgré l'internalisation de l'actif au sein de la nanocapsule, celui-ci a bien été libéré et a pénétré dans le peau pour y exercer son activité tout en étant mieux toléré.

## Revendications

1. Nanocapsule lipidique contenant une phase interne huileuse liquide ou semi-liquide à température ambiante et une enveloppe non polymérique solide ou semi-solide obtenue à partir d'au moins un surfactant choisi parmi les lécithines, **caractérisée en ce qu'**elle contient l'acide 3"-tert-butyl-4'-(2-hydroxy-ethoxy)-4"-pyrrolidin-1-yl-[1, 1';3',1"] terphenyl-4-carboxylique, en tant que principe actif irritant solubilisé, et ce qu'elle ne comprend pas de solvant organique de type alcoolique.

2. Nanocapsule selon la revendication 1, **caractérisée en ce qu'**elle est substantiellement exempte de co-surfactant distinct des phospholipides.

3. Nanocapsule selon l'une des revendications 1 à 2, **caractérisée en ce qu'**elle est substantiellement exempte de polymère.

4. Nanocapsule selon l'une des revendications 1 à 3, **caractérisée en ce que** la phase interne huileuse comprend un corps gras liquide ou semi-liquide à température ambiante.

5. Nanocapsule selon la revendication 1 à 4 **caractérisée en ce que** la phase interne huileuse comprend au moins un solvant huileux de l'actif choisi parmi les acides gras polyéthoxylés, les triglycérides et huiles en contenant et les esters d'acides gras.

6. Nanocapsule selon l'une des revendications 4 et 5, **caractérisée en ce que** la phase interne huileuse est un ester d'acide gras, de préférence le diisopropyl adipate.

7. Nanocapsule selon l'une des revendications 1 à 6, **caractérisée en ce que** le surfactant est choisi parmi les lécithines ayant une quantité en poids de phosphatidylcholine hydrogénée supérieure à 85%.

8. Nanocapsule selon l'une des revendications 1 à 7, **caractérisée en ce qu'**elle présente une taille moyenne inférieure à 900nm, de préférence une taille moyenne inférieure à 500 nm.

9. Nanodispersion comprenant les nanocapsules selon l'une des revendications 1 à 8 précédentes, **caractérisée en ce que** les nanocapsules sont dispersées dans une phase hydrophile.

10. Composition **caractérisé en ce qu'**elle comprend, au sein d'un véhicule pharmaceutiquement acceptable, la nanodispersion définie selon la revendication 9.

11. Composition selon la revendication précédente, pour améliorer la tolérance d'un rétinoïde, en tant que principe actif irritant, comprenant l'acide 3"-tert-butyl-4'-(2-hydroxy-ethoxy)-4"-pyrrolidin-1-yl-[1,1';3',1"] terphenyl-4-carboxylique, en tant que principe actif irritant, **caractérisée en ce qu'**elle comprend, au sein d'un véhicule pharmaceutiquement acceptable, des nanocapsules lipidiques dispersées dans une phase hydrophile, lesdites nanocapsules lipidiques contenant une phase interne huileuse liquide ou semi-liquide à température ambiante dans laquelle le principe actif irritant est solubilisé en l'absence de co-solvant de type alcoolique, et une enveloppe non polymérique solide ou semi-solide obtenue à partir d'au moins un surfactant choisi parmi les lécithines, ladite phase interne huileuse ne contenant pas de solvants de type acides gras non éthoxylé.

12. Composition selon l'une des revendications 10 à 11, **caractérisée en ce que** le véhicule pharmaceutiquement acceptable est un gel, une solution ou une émulsion huile dans eau.

13. Composition selon l'une des revendications 10 à 12, **caractérisée en ce qu'**elle comprend, dans un véhicule pharmaceutiquement acceptable, en poids par rapport au poids total de la composition, des nanocapsules composés de :
a) 0,1 à 5% de surfactant choisi parmi la lécithine ;
b) 1 à 20% d'esters d'acide gras liquide ou semi-liquide à température ambiante ;
c) 0,00001 et 0,1% d'au moins d'acide 3"-tert-butyl-4'-(2-hydroxy-ethoxy)-4"-pyrrolidin-1-yl-[1,1';3',1"]terphenyl-4-carboxylique.

14. Composition selon l'une des revendications 10 à 13, à titre de médicament.

15. Composition telle que définie selon l'une des revendications 10 à 14 pour son utilisation dans le traitement d'affections dermatologiques.

16. Composition telle que définie dans la revendication 15 pour son utilisation dans le traitement d'affections dermatologiques choisies parmi l'acné, les ichtyoses, les états ichtyosiformes, l'hyperkératose palmoplantaire ou le psoriasis.

17. Procédé de préparation d'une composition selon l'une des revendications 10 à 14, **caractérisé en ce qu'**il comprend les étapes suivantes :
(i) Solubilisation de l'acide 3"-tert-butyl-4'-(2-hydroxy-ethoxy)-4"-pyrrolidin-1-yl-[1,1';3',1") terphenyl-4-carboxylique dans un ester d'acide gras liquide ou semi-liquide à température ambiante, afin d'obtenir la phase huileuse ;
(ii) Préparation de la phase hydrophile ;
(iii) Dispersion de la phosphatidylcholine hydrogénée dans la phase huileuse obtenue en (i) ou dans la phase hydrophile obtenue en (ii) ;
(iv) Chauffage des deux phases huileuses et hydrophiles séparément à environ 75°C
(v) Mélange sous agitation des phases huileuse et hydrophile obtenues à l'issue de l'étape (iv) ;
(vi) Introduction du mélange obtenu en (v) dans un Homogénéisateur Haute Pression, afin d'obtenir une composition de nanocapsules lipidiques.
(vii) incorporation de la composition précédente dans un véhicule pharmaceutiquement acceptable.

## Patentansprüche

1. Lipidnanokapsel, die eine ölige flüssige oder halbflüssige innere Phase bei Umgebungstemperatur und eine feste oder halbfeste nicht polymere Umhüllung enthält, die aus mindestens einem aus den Lecithinen ausgewählten Tensid erhalten wird, **dadurch gekennzeichnet, dass** sie die 3"-tert-Butyl-4'-(2-hydroxy-ethoxy)-4"-pyrrolidin-1-yl-[1,1'-3',1"]-Terphenyl-4-carbonsäure als gelösten reizenden Wirkstoff enthält und dass sie kein organisches Lösungsmittel vom alkoholischen Typ enthält.

2. Nanokapsel nach Anspruch 1, **dadurch gekennzeichnet, dass** sie im Wesentlichen frei von Co-Tensiden ist, die sich von Phospholipiden unterscheiden.

3. Nanokapsel nach einem der Ansprüche 1 bis 2, **dadurch gekennzeichnet, dass** sie im Wesentlichen frei von Polymeren ist.

4. Nanokapsel nach einem der Ansprüche 1 bis 3, **dadurch gekennzeichnet, dass** die ölige innere Phase eine flüssige oder halbflüssige Fettsubstanz bei Umgebungstemperatur enthält.

5. Nanokapsel nach Anspruch 1 bis 4, **dadurch gekennzeichnet, dass** die ölige innere Phase mindestens ein öliges Lösungsmittel des Wirkstoffs enthält, das aus polyethoxylierten Fettsäuren, Triglyceriden und diesen enthaltenden Ölen und Fettsäureestern ausgewählt wird.

6. Nanokapsel nach einem der Ansprüche 4 und 5, **dadurch gekennzeichnet, dass** die ölige innere Phase ein Fettsäureester ist, vorzugsweise Diisopropyladipat.

7. Nanokapsel nach einem der Ansprüche 1 bis 6, **dadurch gekennzeichnet, dass** das Tensid aus Lecithinen mit einer Gewichtsmenge an hydriertem Phosphatidylcholin von mehr als 85% ausgewählt wird.

8. Nanokapsel nach einem der Ansprüche 1 bis 7, **dadurch gekennzeichnet, dass** sie eine durchschnittliche Größe von weniger als 900nm aufweist, vorzugsweise eine durchschnittliche Größe von weniger als 500 nm.

9. Nanodispersion, die Nanokapseln nach einem der vorhergehenden Ansprüche 1 bis 8 umfasst, **dadurch gekennzeichnet, dass** die Nanokapseln in einer hydrophilen Phase dispergiert sind.

10. Zusammensetzung, die **dadurch gekennzeichnet ist, dass** sie in einem pharmazeutisch akzeptablen Träger die in Anspruch 9 definierte Nanodispersion enthält.

11. Zusammensetzung nach dem vorhergehenden Anspruch zur Verbesserung der Toleranz eines Retinoids als reizender Wirkstoff, die 3"-tert-Butyl-4'-(2-hydroxy-ethoxy)-4"-pyrrolidin-1-yl-[1,1'-3',1"]-Terphenyl-4-carbonsäure als reizenden Wirkstoff umfasst, **dadurch gekennzeichnet, dass** sie in einem pharmazeutisch akzeptablen Träger Lipidnanokapseln enthält, die in einer hydrophilen Phase dispergiert sind, wobei die besagten Lipidnanokapseln eine ölige flüssige oder halbflüssige innere Phase bei Umgebungstemperatur enthalten, in der der reizende Wirkstoff in Abwesenheit eines Co-Lösungsmittel vom alkoholischen Typ gelöst ist, und eine feste oder halbfeste nicht polymere Umhüllung, die aus mindestens einem aus den Lecithinen ausgewählten Tensid erhalten wird, wobei die besagte ölige innere Phase keine Lösungsmittel vom Typ nicht ethoxylierter Fettsäuren enthält.

12. Zusammensetzung nach einem der Ansprüche 10 bis 11, **dadurch gekennzeichnet, dass** der pharmazeutisch akzeptable Träger ein Gel, eine Lösung oder eine Öl-in-Wasser-Emulsion ist.

13. Zusammensetzung nach einem der Ansprüche 10 bis 12, **dadurch gekennzeichnet, dass** sie in einem pharmazeutisch akzeptablen Träger, bezogen auf das Gesamtgewicht der Zusammensetzung, Nanokapseln enthält, die aus Folgendem zusammengesetzt sind:
a) 0,1 bis 5% Tensid, ausgewählt aus Lecithin;
b) 1 bis 20% Fettsäureester, die bei Umgebungstemperatur flüssig oder halbflüssig sind;
c) 0,00001 und 0,1% von mindestens 3"-tert-Butyl-4'-(2-hydroxy-ethoxy)-4"-pyrrolidin-1-yl-[1,1';3',1"]-Terphenyl-4-carbonsäure.

14. Zusammensetzung nach einem der Ansprüche 10 bis 13 als Medikament.

15. Zusammensetzung nach einem der Ansprüche 10 bis 14 zur Verwendung bei der Behandlung dermatologischer Erkrankungen.

16. Zusammensetzung nach Anspruch 15 zur Verwendung bei der Behandlung dermatologischer Erkrankungen, ausgewählt aus Akne, Ichthyosen, ichthyosiformen Zuständen, palmoplantarer Hyperkeratose oder Psoriasis.

17. Verfahren zur Herstellung einer Zusammensetzung nach einem der Ansprüche 10 bis 14, **gekennzeichnet dadurch, dass** es die folgenden Schritte umfasst:
(i) Solubilisierung der 3"-tert-Butyl-4'-(2-hydroxy-ethoxy)-4"-pyrrolidin-1-yl-[1,1';3',1"]-Terphenyl-4-carbonsäure in einem flüssigen oder halbflüssigen Fettsäureester bei Umgebungstemperatur, um die ölige Phase zu erhalten;
(ii) Herstellung der hydrophilen Phase;
(iii) Dispersion des hydrierten Phosphatidylcholins in der unter (i) erhaltenen öligen Phase oder in der unter (ii) erhaltenen hydrophilen Phase;
(iv) Separates Erhitzen der beiden öligen und hydrophilen Phasen auf etwa 75°C;
(v) Mischung der nach Abschluss von Schritt (iv) erhaltenen öligen und hydrophilen Phasen unter Rühren;
(vi) Einbringen der unter (v) erhaltenen Mischung in einen Hochdruckhomogenisator, um eine Zusammensetzung von Lipidnanokapseln zu erhalten.
(vii) Einarbeitung der vorherigen Zusammensetzung in einen pharmazeutisch akzeptablen Träger.

## Claims

1. Lipid nanocapsule containing an oily internal phase which is liquid or semi-liquid at room temperature and a solid or semi-solid non-polymeric shell obtained from at least one surfactant selected from lecithins, **characterized in that** it contains 3"-tert-butyl-4'-(2-hydroxy-ethoxy)-4"-pyrrolidin-1-yl-[1,1';3',1"] terphenyl-4-carboxylic acid as a solubilized irritant active principle, and **in that** it does not comprise an alcoholic organic solvent.

2. Nanocapsule according to claim 1, **characterized in that** it is substantially free of co-surfactants other than phospholipids.

3. Nanocapsule according to one of claims 1 to 2, **characterized in that** it is substantially free of polymers.

4. Nanocapsule according to one of claims 1 to 3, **characterized in that** the oily internal phase comprises a fatty substance which is liquid or semi-liquid at room temperature.

5. Nanocapsule according to claim 1 to 4, **characterized in that** the oily internal phase comprises at least one oily solvent for the active principle selected from polyethoxylated fatty acids, triglycerides and oils containing them and fatty acid esters.

6. Nanocapsule according to one of claims 4 and 5, **characterized in that** the oily internal phase is a fatty acid ester, preferably diisopropyl adipate.

7. Nanocapsule according to one of claims 1 to 6, **characterized in that** the surfactant is selected from lecithins having a hydrogenated phosphatidylcholine content by weight greater than 85%.

8. Nanocapsule according to one of claims 1 to 7, **characterized in that** it has an average size of less than 900 nm, preferably an average size of less than 500 nm.

9. Nanodispersion comprising the nanocapsules according to one of the preceding claims 1 to 8, **characterized in that** the nanocapsules are dispersed in a hydrophilic phase.

10. Composition **characterized in that** it comprises, in a pharmaceutically acceptable carrier, the nanodispersion defined according to claim 9.

11. Composition according to the preceding claim, for improving the tolerance of a retinoid, as an irritant active principle, comprising 3"-tert-butyl-4'-(2-hydroxy-ethoxy)-4"-pyrrolidin-1-yl-[1,1';3',1"] terphenyl-4-carboxylic acid, as an irritant active principle, **characterized in that** it comprises, in a pharmaceutically acceptable carrier, lipid nanocapsules dispersed in a hydrophilic phase, said lipid nanocapsules containing an oily internal phase which is liquid or semi-liquid at room temperature in which the irritant active principle is dissolved in the absence of an alcoholic co-solvent, and a solid or semi-solid non-polymeric shell obtained from at least one surfactant selected from lecithins, said oily internal phase not containing non-ethoxylated fatty acid type solvents.

12. Composition according to one of claims 10 to 11, **characterized in that** the pharmaceutically acceptable carrier is a gel, a solution or an oil-in-water emulsion.

13. Composition according to one of claims 10 to 12, **characterized in that** it comprises, in a pharmaceutically acceptable carrier, by weight relative to the total weight of the composition, nanocapsules composed of:
a) 0.1 to 5% surfactant selected from lecithin;
b) 1 to 20% fatty acid esters which are liquid or semi-liquid at room temperature;
c) 0.00001 and 0.1% of at least 3"-tert-butyl-4'-(2-hydroxy-ethoxy)-4"-pyrrolidin-1-yl-[1.1';3',1"] terphenyl-4-carboxylic acid.

14. Composition according to one of claims 10 to 13, as a drug.

15. Composition as defined according to one of claims 10 to 14 to be used in the treatment of dermatological conditions.

16. Composition as defined in claim 15 to be used in the treatment of dermatological conditions selected from acne, ichthyosis, ichthyosiform conditions, palmoplantar hyperkeratosis or psoriasis.

17. Process for preparing a composition according to one of claims 10 to 14, **characterized in that** it comprises the following steps:
(i) Solubilizing 3"-tert-butyl-4'-(2-hydroxy-ethoxy)-4"-pyrrolidin-1-yl-[1,1';3',1"] terphenyl-4-carboxylic acid in a fatty acid ester which is liquid or semi-liquid at room temperature, in order to obtain the oily phase;
(ii) Preparing the hydrophilic phase;
(iii) Dispersing the hydrogenated phosphatidylcholine in the oily phase obtained in (i) or in the hydrophilic phase obtained in (ii);
(iv) Heating the two oily and hydrophilic phases separately to about 75 °C;
(v) Mixing under stirring the oily and hydrophilic phases obtained at the end of step (iv);
(vi) Introducing the mixture obtained in (v) into a High Pressure Homogenizer, in order to obtain a lipid nanocapsule composition.
(vii) Incorporating the preceding composition into a pharmaceutically acceptable carrier.
